Europäisches Patentamt

European Patent Office

Office européen des brevets

(11) Veröffentlichungsnummer: **0 152 948 B1**

(12) **EUROPÄISCHE PATENTSCHRIFT**

(45) Veröffentlichungstag der Patentschrift: **08.05.91**

(21) Anmeldenummer: **85101845.7**

(22) Anmeldetag: **20.02.85**

(51) Int. Cl.⁵: **C12P 17/12, C12N 1/20,**
**//(C12N1/20,C12R1:025),**
**(C12P17/12,C12R1:025)**

(54) Verfahren zur Herstellung von 6-Hydroxynikotinsäure.

(30) Priorität: **21.02.84 CH 825/84**

(43) Veröffentlichungstag der Anmeldung:
**28.08.85 Patentblatt 85/35**

(45) Bekanntmachung des Hinweises auf die
Patenterteilung:
**08.05.91 Patentblatt 91/19**

(84) Benannte Vertragsstaaten:
**AT BE CH DE FR GB IT LI NL SE**

(56) Entgegenhaltungen:
**EP-A- 0 152 949**

**CHEMICAL ABSTRACTS, Band 61, Nr. 2, 20.
Juli 1964, Spalte 2208-g, Columbus, Ohio, US;
J.C. ENSIGN et al.; "The pathway of nicotinic
acid oxidation by a Bacillus species"**

**CHEMICAL ABSTRACTS, Band 47, Nr. 6, 25.
März 1953, Spalte 2832h-i, Columbus, Ohio,
US; D.E. HUGHES: "Hydroxynicotinic acid as
intermediate in oxidation of nicotinic acid by
Pseudomonas fluorescens"**

(73) Patentinhaber: **LONZA AG**

**Gampel/Wallis(CH)**

(72) Erfinder: **Lehky, Pavel, Dr.**
**Dammweg 13**
**CH-Naters (Kanton Wallis)(CH)**
Erfinder: **Kulla, Hans, Dr.**
**Wildi**
**CH-Visp (Kanton Wallis)(CH)**
Erfinder: **Mischler, Stephane, Dr.**
**Rathausstrasse 12**
**CH-Visp (Kanton Wallis)(CH)**

(74) Vertreter: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

CHEMICAL ABSTRACTS, Band 54, Nr. 20, 25. Oktober 1960, Spalte 21307b-c, Columbus, Ohio, US; T. SUGAHARA: "Microbial degradation of nicotinic acid and nicotinamide", BI-TAMIN 14, 245-8(1958)

## Beschreibung

Die Erfindung betrifft ein Verfahren zur Herstellung von 6-Hydroxynikotinsäure durch enzymatische Hydroxylierung von Nikotinsäure.

Mehrere Methoden zur Herstellung von 6-Hydroxynikotinsäure mittels organischer Synthesen sind bekannt: aus 2-Pyridon kann man die 6-Hydroxynikotinsäure durch die Kolbe-Schmidtische Carboxylierung von Hydroxyaromaten erhalten. Andere Synthesen gehen von Apfelsäure oder Isocinchomeronsäure aus [Briancourt et al., J.Chim.Ther. (1973), 8 (2) 226-32; Quarroz, CH-Anm. Nr. 7731/80).

Keine dieser bekannten Synthesen erlaubt eine einfache, billige und umweltfreundliche Herstellung von reiner 6-Hydroxynikotinsäure. Den Verfahren haftet der Nachteil an, dass die Umsetzung nicht quantitativ ist und unerwünschte Nebenprodukte die Reaktion begleiten. Die Nebenprodukte stellen eine Verunreinigung dar und müssen nach beendeter Reaktion aus dem Reaktionsprodukt entfernt werden.

Es ist auch bekannt, dass Mikroorganismen der Gattungen Bacillus, Pseudomonas, Clostridium, Sarcina und Mycobacterium auf Nikotinsäure wachsen und dass sie dieses Substrat als Kohlenstoff-, Stickstoff- und Energiequelle benutzen [Allinson M.J.C.:J.Biol.Chem. (1943) 147, 785; Behrman E.J. et Stanier R.V., J.Biol.Chem. (1957) 228, 923].

Die Nikotinsäure wird bei allen studierten Organismen im ersten Abbauschritt zu 6-Hydroxynikotinsäure oxidiert. Die 6-Hydroxynikotinsäure wird sofort und ohne bedeutende Anreicherung weiter umgewandelt, bei aeroben Organismen bis zu Wasser, Kohlendioxid und Ammoniak.

Nach Aufbrechen der Mikroorganismen war es möglich, die Nikotinsäure-Hydroxylase in mehr oder weniger reiner Form zu isolieren [Hunt A.L., Biochem.J. (1958) 72, 1-7]. Die Nikotinsäure-Hydroxylasen sind grosse Moleküle von etwa 400'000 Dalton. Sie enthalten Flavinkofaktoren, viele Metallatome (Fe, Mo), anorganischen Schwefel und in einigen Fällen sogar Selenium. Die Nikotinsäure-Hydroxylasen sind aktiv nur in Anwesenheit der geeigneten elektronübertragenden Systeme (z.B. Cytochrome, Flavine, $NADP^+$ und andere).

Es ist möglich, die Nikotinsäure-Hydroxylase aus Zell-Extrakten zu isolieren und die Enzym-Präparate zur Hydroxylierung der Nikotinsäure anzuwenden. Dies wurde gemacht und kleine Mengen von 6-Hydroxynikotinsäure wurden tatsächlich erhalten [Behrman u. Stanier, J.Biol.Chem. (1957) 228, 923]. Abgesehen von den hohen Enzym-Isolierungskosten und der Unstabilität der Nikotinsäure-Hydroxylase musste man noch für die Regenerierung von Kofaktoren und Elektronenübertragungs-Systeme sorgen.

Aufgabe vorliegender Erfindung war es, diese Nachteile zu überwinden und ein Verfahren vorzuschlagen, das es ermöglicht, auf wirtschaftliche Weise aus Nikotinsäure die 6-Hydroxynikotinsäure in höchster Reinheit und Ausbeute herzustellen.

Erfindungsgemäss wird das mit einem Verfahren nach Patentanspruch 1 erreicht.

Es wurde gefunden, dass Mikroorganismen der Gattungen Pseudomonas, Bacillus und Achromobacter die Herstellung der 6-Hydroxynikotinsäure mit Erfolg durchführen lässt: Zweckmässig wird der Achromobacter xylosoxydans DSM 2402, Pseudomonas putida NCIB 10521 oder ein Bacillus-Stamm, der von Ensign und Rittenberg [J.Biol. Chem. 239, (1964) 2285-2291] beschrieben wurde, angewendet, vorzugsweise wird der Achromobacter xylosoxydans DSM 2783 angewendet.

Vorzugsweise wird der neue Stamm Achromobacter xylosoxydans DSM 2783 angewendet.

| | |
|---|---|
| Name: | Achromobacter xylosoxydans DSM Nr. 2783 |
| Isoliert aus: | Nikotinsäure-Mutterlauge |

(A) Morphologie

Kultivierung in Nutrient-Broth
(1) Zellform:
Stäbchen 2-3,5 $\mu$m lang, etwa 0,6 $\mu$m breit
(2) Anordnung:
einzeln
(3) Motilität:
stark beweglich; peritrich begeisselt
(4) Endospore:
strikt aerob
(5) Gram:
negativ
(6) Oxidaxe:
positiv
(7) Catalase:
positiv

Der Stamm stimmt in allen geprüften Merkmalen mit dem Typstrain von Achromobacter xylosoxydans (DSM 2402) überein (Ausnahme: Hydrolyse von Acetamid).

Die genannten Stämme sind bei der Deutschen Sammlung von Mikroorganismen (DSM), Gesellschaft für Biotechnologische Forschung mbH., Griesebachstrasse 8, 4300 Göttingen, Bundesrepublik Deutschland, unter der Nr. DSM 2402 und DSM 2783 deponiert.
Der neue Stamm DSM 2783 wurde an der oben genannten Hinterlegungsstelle am 18.11.1983 hinterlegt.

Die Stämme Pseudomonas putida NCIB 10521 und 8176 sind bei der National Collection of Industrial Bacteria, Torry Research Station, 135 Abbey Road, Aberdeen AB98DC, Scotland, erhältlich.

Die genannten Stämme wachsen mit Nikotinsäure als einzige Kohlenstoff-, Stickstoff- und Energiequelle.

Die Züchtung der genannten Mikroorganismen kann nach den für diese Art von Stämmen bekannten Verfahren erfolgen.

Beispielsweise wird der Stamm DSM 2783 in einer verdünnten und sterilisierten Nikotinsäure-Lösung (0,05-0,5 Gew.%), die Phosphat-Puffer (50mM) pH 7,0,

Spurenelemente (in mg/l)

| $CaCl_2 . 2H_2O$ | 20 |
|---|---|
| $MnSO_4$ | 10 |
| $FeSO_4 . 7H_2O$ | 5 |
| $CoCl_2 . 6H_2O$ | 0,1 |
| $CuSO_4 . 5H_2O$ | 0,1 |
| $ZnSO_4 . 7H_2O$ | 0,1 |
| $NaMoO_4 . 2H_2O$ | 0,1 |

und, um das Wachstum zu beschleunigen, eine kleine Menge Hefeextrakt (Merck) (0,05 Gew.%) enthält, während 24 bis 48 Std. bei 30°C unter aeroben Bedingungen fermentiert.

Die gewachsene Biomasse (etwa 10 g Feuchtgewicht/1) ist reich an Nikotinsäure-Hydroxylase. Die Zellen werden abzentrifugiert und können sofort oder nach Lagerung bei -20°C direkt, d.h. ohne Enzymgewinnung oder Reinigung, für die Nikotinsäure-Hydroxylierung eingesetzt werden.

Für die Durchführung der Nikotinsäure-Hydroxylierung ist es wünschenswert, dass der Abbau der Nikotinsäure nicht über den ersten Schritt, nämlich die Hydroxylierung zur 6-Hydroxynikotinsäure, hinausgeht. In dieser Produktionsphase würde das Wachstum des Mikroorganismus auf Kosten der Ausbeute gehen.

Die folgenden, für die Wirtschaftlichkeit der Nikotinsäure-Hydroxylierung wichtigen Parameter müssen erfüllt werden:

a) die Zellen sollten nicht mehr wachsen (Verbrauch von Nikotinsäure),

b) die Nikotinsäure-Hydroxylase soll aktiv bleiben,

c) der Abbauweg der Nikotinsäure sollte auf der Stufe der 6-Hydroxynikotinsäure abgebrochen werden,

d) das Produkt (6-Hydroxynikotinsäure) soll aus der Zelle ausgeschieden werden.

Es wurde überraschenderweise gefunden, dass diese Parameter gleichzeitig erfüllt sind, wenn die Konzentration der Nikotinsäure erhöht wird. Dieses für die Herstellung der 6-Hydroxynikotinsäure günstige Verhalten scheint im mikrobiellen Metabolismus weit verbreitet zu sein. Es ist anzunehmen, dass das Folge-Enzym (6-Hydroxynikotinsäure-Hydroxylase) durch hohe Nikotinsäure-Konzentrationen gehemmt wird.

Wie oben erwähnt, wächst der Stamm DSM 2783 vorzugsweise in verdünnten Nikotinsäure-Lösungen (0,05-0,5 Gew.%) und verbraucht die vorgelegte Nikotinsäure dabei vollständig. Mit steigender Konzentration der Nikotinsäure wird das Zellwachstum gehemmt und oberhalb 2 Gew.% Nikotinsäure-Konzentration kann kein Wachstum mehr beobachtet werden. Die Aktivität der Nikotinsäure-Hydroxylase jedoch bleibt in den Zellen unverändert.

Die Reaktion der enzymatischen Hydroxylierung läuft vorteilhaft bei 20 bis 40°C und einem PH von 5,5 bis 9,0 unter aeroben Bedingungen ab. Zweckmässig werden 0,1 Gew.%ige bis gesättigte, vorzugsweise 0,5 bis 10 Gew.%ige, Nikotinsäurelösungen angewendet. Die Nikotinsäure kann auch in Form der Alkalisalzlösungen eingesetzt werden.

Der katabolische Abbau wird nach der Hydroxylierungsstufe unterbrochen. Deshalb sind die Neben- und Folgereaktionen eliminiert und die Reinheit und Ausbeute der 6-Hydroxynikotinsäure sind sehr hoch.

Eine weitere, positive Eigenschaft der untersuchten Mikroorganismen ist, dass sie das Produkt der Hydroxylierung, die 6-Hydroxynikotinsäure, in die Lösung ausscheiden. Das vereinfacht die Isolierung des Produktes wesentlich.

Nach dem Abtrennen von Zellen aus der Reaktionsbrühe durch Zentrifugation oder Mikrofiltration wird die klare Lösung angesäuert. Die dabei ausgefallene, weisse 6-Hydroxynikotinsäure wird abfiltriert und getrocknet.

Beispiele

Aus praktischen Erwägungen wurde die labormässige Herstellung der 6-Hydroxynikotinsäure für eine einmolare Menge in zwei Schritte getrennt:

Schritt 1: Herstellung der Biomasse mit hoher NS-Hydroxylaseaktivität,

Schritt 2: Enzymatische Hydroxylierung der Nikotinsäure.

Es ist natürlich ohne weiteres möglich, die beiden Schritte zu einem sogenannten Eintopf-Verfahren zu kombinieren.

Beispiel 1

(Schritt 1) Herstellung der Achromobacter xylosoxydans DSM 2783 Biomasse.

Eine Nährlösung, die 51,9 g $Na_2HPO_4.2H_2O$, 20,0 g $KH_2PO_4$, 2,5 g Hefeextrakt und 10 g Nikotinsäure in 4750 ml enthielt, wurde in den Fermentor eingefüllt und 20 Min. bei 120°C sterilisiert. Nach Abkühlen auf 30°C wurde eine sterile Lösung von oben beschriebenen Spurenelementen zugegeben, der Fermentor mit 500 ml der einer Starter-Kultur (gleiche Zusammensetzung) angeimpft und bei 30°C, pH 7,0, unter Begasung mit Luft 24 Std. fermentiert. Nach 24 Std. wurden 200 ml einer Lösung von 10 g Nikotinsäure und 2,5 g Hefeextrakt in Wasser steril zugegeben und die Fermentation weitergeführt. Nach 42 Std. wurde die Kultur geerntet und die Zellen durch Zentrifugation (30 Min. bei 15'000) getrennt.

Man erhielt 38,3 g feuchte Biomasse.

(Schritt 2) Hydroxylierung der Nikotinsäure.

Das 3 l-Reaktionsgefäss wurde mit 2250 ml 5%iger Natrium-nikotinat-Lösung (pH 6,5) gefüllt und auf 30°C aufgewärmt. Die Suspension von DSM 2783-Zellen in 120 ml Wasser wurde zugegeben und die Reaktionsmischung bei gutem Rühren intensiv belüftet. Der pH, die Temperatur und die Sauerstoffkonzentration in der Reaktionsmischung wurden kontinuierlich gemessen und geregelt. Nach 7 Std. stieg die Konzentration des gelösten Sauerstoffes an. Zu diesem Zeitpunkt war die Reaktion beendet. Die Reaktionssuspension wurde abzentrifugiert und die Zellen im Sediment für die nächste Charge verwendet.

Der klare Ueberstand wurde mit konz. Schwefelsäure auf pH 1,5 gebracht und die ausgefallene 6-Hydroxynikotinsäure abgenutscht und getrocknet.

Man erhielt 121 g eines weissen Produktes, das laut HPLC-Analyse 98,6% 6-Hydroxynikotinsäure enthielt. Dies entsprach einer 6-Hydroxynikotinsäure-Ausbeute von 93,7%, bezogen auf die eingesetzte Nikotinsäure.

### Beispiel 2

(Schritt 1) Herstellung der Pseudomonas putida NCIB 10521-Biomasse.

1 l der gleichen Nährlösung, die in Beispiel 1 beschrieben ist, wurde in einem 2 l-Kolben sterilisiert und nach Abkühlen auf 30°C mit einer Pseudomonas-Kultur NCIB 10521 von einer Agar-Platte angeimpft. Die Kultur wurde 48 Std bei 30°C im Brutschrank geschüttelt. Nach Erreichen der maximalen Zell-Dichte wurden die Zellen während 20 Min bei 10'000 g abzentrifugiert. Die Zellen wurden in 10 ml Phosphatpuffer (50 mM, pH 7) aufgeschlämmt.

(Schritt 2)

Ein 1 l-Schüttel-Kolben wurde mit 100 ml neutraler 40 mM - Natriumnikotinat-Lösung gefüllt und 10 ml Zell-Suspension (aus Schritt 1) zugegeben. Die Mischung wurde 90 Min im Brutschrank bei 30°C intensiv geschüttelt. Die Zellen wurden abzentrifugiert und der klare Ueberstand (108 ml) für Nikotin- und 6-Hydroxynikotinsäure analysiert.

Laut HPLC-Analyse enthielt die Lösung 36,1 mM 6-Hydroxynikotinsäure (Na-Salz). Dies entspricht einer Ausbeute von 97,5%, auf die eingesetzte Nikotinsäure berechnet.

Die Konzentration der Nikotinsäure war tiefer als 0,2% der 6-Hydroxynikotinsäure-Konzentration.

Die feste 6-Hydroxynikotinsäure konnte aus der Lösung durch Ansäuern mit einer starken Säure isoliert werden.

### Beispiel 3

(Schritt 1) Herstellung der Pseudomonas Putida NCIB 8176-Biomasse.

Die Pseudomonas-Zellen wurden, wie in Beispiel 1, Schritt 1, fermentiert. Man erhielt 45,3 g feuchte Biomasse.

(Schritt 2) Hydroxylierung der Nikotinsäure.

Das 3 l-Reaktionsgefäss wurde mit 1125 ml 10%iger Natriumnikotinat-Lösung (pH 7,0) gefüllt und auf 35°C aufgewärmt. Die Suspension von NCIB 8176-Biomasse in 100 ml Wasser wurde zugegeben und die Reaktionsmischung bei gutem Rühren intensiv belüftet. Der pH, die Temperatur und die Sauerstoffkonzentration in der Reaktionsmischung wurden kontinuierlich gemessen und geregelt (pH = 7,0; Temp. = 35°C; $pO_2$ = 5 mg/l). Nach 5 Std und 20 Min stieg die Konzentration des gelösten Sauerstoffes in einem Sprung auf 7 mg/l. Zu diesem Zeitpunkt war die Reaktion beendet. Die Reaktionssuspension wurde mit Amicon Hollow-Fiber H1MPO1-43 Filter filtriert. Die stark konzentrierte (50 x) Zell-Suspension wurde für die nächste Charge verwendet.

Der klare Ueberstand wurde mit konzentrierter Salzsäure auf pH 1,5 gebracht und die ausgefallene schneeweisse 6-Hydroxynikotinsäure abgenutscht, auf dem Filter mit Wasser gewaschen und im Vakuum (20 mbar; 60°C; 10 Std) getrocknet.

Man erhielt 122,3 g eines weissen Produktes, das laut HPLC-Analyse 99,2% 6-Hydroxynikotinsäure enthielt. Dies entspricht einer 6-Hydroxynikotinsäure-Ausbeute von 95,4%, bezogen auf die eingesetzte Nikotinsäure.

### Ansprüche

1.  Verfahren zur Herstellung von 6-Hydroxynikotinsäure durch Hydroxylierung von Nikotinsäure, dadurch gekennzeichnet, daß die Hydroxylierung enzymatisch in Gegenwart eines Nikotinsäure abbauenden Mikroorganismus der Gattung Pseudomonas, Bacillus oder Achromobacter ausgeführt wird und die Konzentration an Nikotinsäure so gewählt wird, daß der Abbau der Nikotinsäure auf der Stufe der 6-Hydroxynikotinsäure gehemmt wird.

2. Verfahren gemäss Patentanspruch 1, dadurch gekennzeichnet, dass ein Achromobacter xylosoxydans angewendet wird.

3. Verfahren gemäß Patentansprüchen 1 und 2, dadurch gekennzeichnet, dass ein Achromobacter xylosoxydans mit der Bezeichnung DSM 2783 oder eine wirksame Mutante von diesem angewendet wird.

4. Verfahren gemäß Patentansprüchen 1 bis 3, dadurch gekennzeichnet, dass Pseudomonas putida NCIB 10521 bzw. 8176 oder eine wirksame Mutante von diesen angewendet wird.

5. Verfahren gemäß Patentansprüchen 1 bis 4, dadurch gekennzeichnet, dass die enzymatische Hydroxylierung bei 20 bis 40°C und pH 5,5 bis 9,0 unter aeroben Bedingungen ausgeführt wird.

6. Verfahren gemäß Patentansprüchen 1 bis 5, dadurch gekennzeichnet, dass eine 0,1 Gew.%ige bis gesättigte, vorzugsweise eine 0,5 bis 10 Gew.%ige, Nikotinsäurelösung bzw. die Lösung einer entsprechenden Menge an Alkalinikotinat angewendet wird.

7. Achromobacter xylosoxydans DSM 2783 und seine die Fähigkeit zur spezifischen enzymatischen Hydroxylierung von Nikotinsäure zur 6-Hydroxynikotinsäure aufweisenden Mutanten.

## Claims

1. Process for the preparation of 6-hydroxynicotinic acid by hydroxylation of nicotinic acid, characterized in that the hydroxylation is carried out enzymatically in the presence of a microorganism which is capable of degrading nicotinic acid and belonging to the genera Pseudomonas, Bacillus or Achromobacter and that the concentration of nicotinic acid is chosen so that the degradation of nicotinic acid is inhibited at the stage of 6-hydroxynicotinic acid.

2. Process according to patent claim 1, characterized in that Achromobacter xylosoxydans is used.

3. Process according to patent claims 1 and 2, characterized in that Achromobacter xylosoxydans with the designation DSM 2783 or an effective mutant thereof is used.

4. Process according to patent claims 1 to 3, characterized in that Pseudomonas putida NCIB 10521 and 8176 resp. or an effective mutant thereof is used.

5. Process according to patent claims 1 to 4, characterized in that the enzymatic hydroxylation is carried out at 20 to 40°C and at a pH of 5.5 to 9.0 under aerobic conditions.

6. Process according to patent claims 1 to 5, characterized in that a nicotinic acid solution of a concentration between 0.1 weight% and saturation, preferably between 0.5 and 10 weight%, or a solution of the corresponding amount of an alkali nicotinate is used.

7. Achromobacter xylosoxydans DSM 2783 and its mutants capable of the specific enzymatic hydroxylation of nicotinic acid to 6-hydroxynicotinic acid.

## Revendications

1. Procédé de préparation de l'acide 6-hydroxynicotinique par hydroxylation de l'acide nicotinique, caractérisé en ce que l'hydroxylation est effectuée enzymatiquement en présence d'un micro-organisme dégradant l'acide nicotinique, de l'espèce Pseudomonas, Bacillus ou Achromobacter et en ce que la concentration en acide nicotinique est choisie de telle sorte que la dégradation de l'acide nicotinique est inhibée à l'étape de l'acide 6-hydroxynicotinique.

2. Procédé selon la revendication 1, caractérisé en ce qu'on utilise un Achromobacter xylosoxydans.

3. Procédé selon les revendications 1 et 2, caractérisé en ce que l'on utilise un Achromobacter xylosoxydans ayant la dénomination DSM 2783 ou un mutant actif de celui-ci.

4. Procédé selon les revendications 1 à 3, caractérisé en ce que l'on utilise Pseudomonas putida NCIB 10521 ou 8176 ou un mutant actif de ceux-ci.

5. Procédé selon les revendications 1 à 4, caractérisé en ce que l'hydroxylation enzymatique est effectuée à 20 jusqu'à 40°C et à pH 5,5 jusqu'à 9,0 dans des conditions aérobies.

6. Procédé selon les revendications 1 à 5, caractérisé en ce qu'on utilise une solution d'acide nicotinique à 0,1 % en poids jusqu'à la saturation, de préférence une solution d'acide nicotinique à 0,5 jusqu'à 10 % en poids ou la solution d'une quantité correspondante d'un nicotinate alcalin.

7. Achromobacter xylosoxydans DSM 2783 et ses mutants présentant l'aptitude à l'hydroxylation enzymatique spécifique de l'acide nicotinique en acide 6-hydroxynicotinique.